Europäisches Patentamt

European Patent Office

Office européen des brevets

(11) Publication number: **0 194 105 B1**

## (12) EUROPEAN PATENT SPECIFICATION

(45) Date of publication of patent specification: **02.05.91**

(51) Int. Cl.⁵: **G01N 21/31**, A61B 5/00, H05B 33/08

(21) Application number: **86301425.4**

(22) Date of filing: **27.02.86**

(54) Oximeter.

(30) Priority: **28.02.85 US 706406**

(43) Date of publication of application:
**10.09.86 Bulletin 86/37**

(45) Publication of the grant of the patent:
**02.05.91 Bulletin 91/18**

(84) Designated Contracting States:
**DE FR GB NL**

(56) References cited:
**EP-A- 0 102 816**
**WO-A-82/03322**
**DE-A- 2 136 823**
**US-A- 4 295 137**
**US-A- 4 446 871**

(73) Proprietor: **THE BOC GROUP, INC.**
**575 Mountain Avenue**
**Murray Hill, New Providence, New Jersey**
**07974(US)**

(72) Inventor: **Nichols, Robert A.**
**4301 East 107 Court**
**Thorton Colorado 80233(US)**
Inventor: **Pologe, Jonas A.**
**515 Hartford Drive**
**Boulder Colorado 80303(US)**

(74) Representative: **Dipl.-Phys.Dr. Manitz**
**Dipl.-Ing.Dipl.-Wirtsch.-Ing. Finsterwald**
**Dipl.-Phys. Rotermund Dipl.-Chem.Dr. Heyn**
**B.Sc.(Phys.) Morgan**
**Robert-Koch-Strasse 1**
**W-8000 München 22(DE)**

Description

This invention is directed to an oximeter of the type including a probe for transmitting light of two different frequencies to a photosensing diode, by way of living tissue, in order to ascertain the oxygen saturation of blood in the tissue.

An oximeter of this type is disclosed in U.S. Patent No. 4,407,290, to Wilber, assigned to the assignee of the present application.

The Wilber patent discloses various algorithms employed in the determination of oxygen saturation in an oximeter of the above type. In the system specifically disclosed in the Wilber patent, however, a normalization procedure is effected by the provision of a hardware feedback of signals.

Further, the sampling of signals is synchronized with the AC power frequency in order to avoid power frequency interference, means being provided to determine the frequency employed and to control the timing circuit in accordance therewith.

According to the present invention an oximeter including means for transmitting light of at least two different wavelengths through blood-containing tissue, sensing means for transducing said transmitted light of said different wavelengths into signals, means for producing a plurality of signals corresponding to the attenuation of said transmitted light at the different wavelengths, data processing means responsive to said signals for determining the oxygen saturation level SaO$_2$ of blood in said tissue, and digital display means for displaying said oxygen saturation level; is characterised in that said display means further comprises an LCD matrix display, said data processing means including means for applying signals to the matrix display for displaying a plethysmographic waveform and further means for controlling said matrix display to display thereon a bar having an amplitude corresponding to the average amplitude of the AC component of said signals.

An embodiment of the invention will now be described by way of example with reference to the Figures of the accompanying diagrammatic drawings in which:-

Figure 1 is a block diagram of an analog portion of an oximeter in accordance with the invention;

Figure 2 is a circuit diagram of a timing circuit for the analog circuit of Figure 1;

Figure 3 is a block diagram of a digital control circuit for the oximeter;

Figure 4 is a circuit diagram of the AC power frequency detector for use in the oximeter of the invention;

Fig. 5 is a block diagram of the display;

Fig. 6 is a circuit diagram of a variable pitch alarm circuit;

Fig. 7 is a circuit diagram of a visual alarm for the system;

Fig. 8 is a plan view of the panel of an oximeter in accordance with the invention.

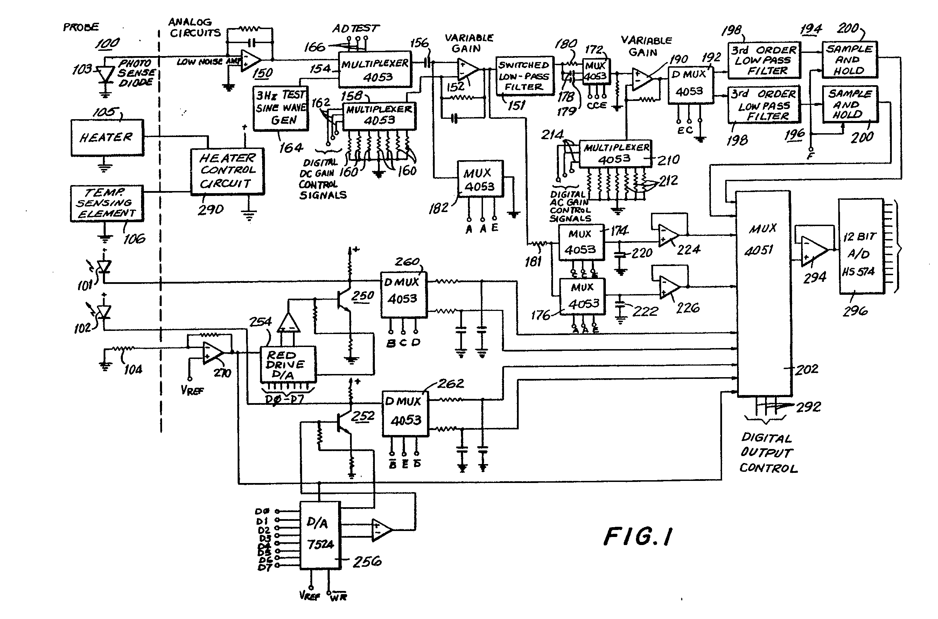

Referring now to the drawings, Fig. 1 generally illustrates the analog portions of an oximeter in accordance with the invention. The probe 100 is comprised of a red LED 101, an infra-red LED 102, and photo-sensing diode 103 adapted to receive light from the red and infra-red LEDs 101, 102. In addition, the probe 100 includes a resistor 104 having a resistance arbitrarily corresponding to the characteristics of the probe, a heater 105 such as a resistive heater for heating the probe to a constant temperature, and a temperature-sensing element 106 for sensing the temperature of the probe.

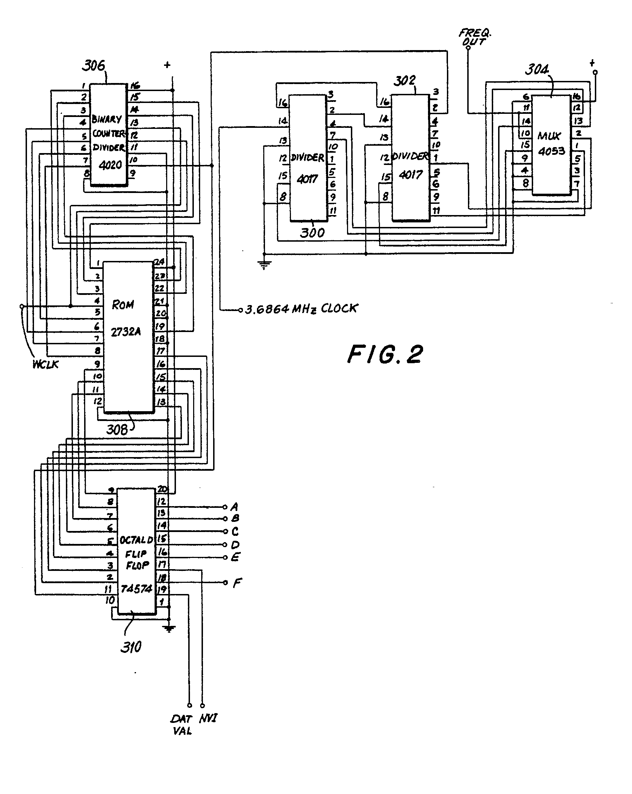

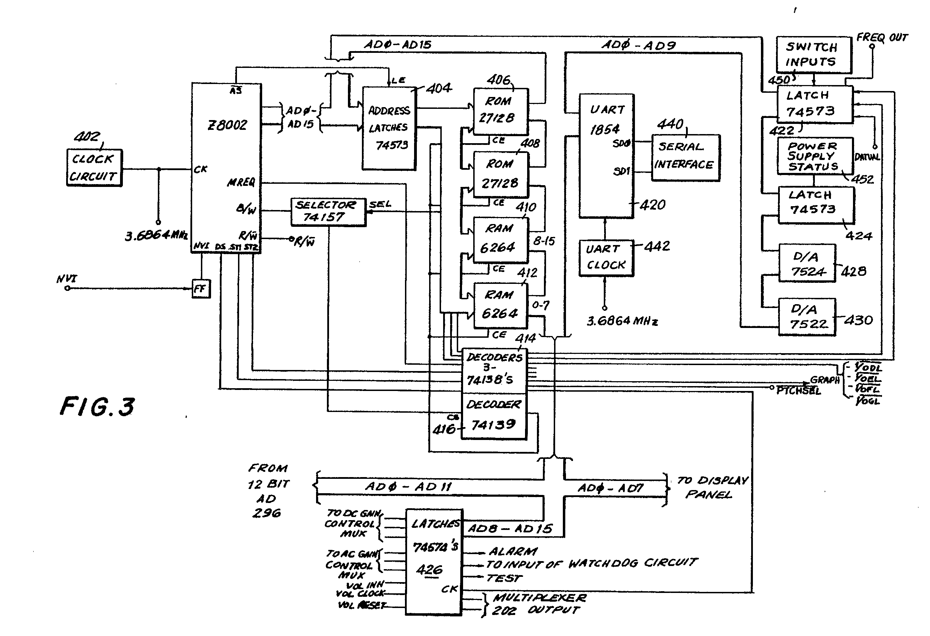

The signal output of the photo-sensing diode 103, corresponding to light received from the red and infra-red LEDs 101, 102, as well as ambient light, is applied to a low-noise amplifier 150. These signals may thus have a DC level which is dependent upon the ambient light and the light absorbed by the blood in the tissue being sampled, and an AC component corresponding to variations in density resulting from arteriole blood pulsations. The signal output of the low-noise amplifier 150 is applied to a variable gain amplifier 152 by way of a multiplexer 154 and series capacitor 156. The gain of the amplifier 152 may be controlled by multiplexer 158 connected to selectively apply different input resistors 160 to the amplifier, the multiplexer being in turn controlled by control lines 162, to which digital control signals are applied from the digital portion of the system as will be discussed with reference to Fig. 3. The multiplexer 154 is adapted also to apply selectively test signals to the variable gain amplifier 152 from a 3 Hz sine wave generator 164, for simulating input signals to test the apparatus. The multiplexer 154 is controlled by the timing circuit of Fig. 2, the control signals thereof being applied to the control lines 166 of the multiplexer 154. The output of the amplifier 152, which still contains AC and DC components, is applied via the switched low-pass filter 151 to multiplexers 172, 174 and 176, the inputs of the multiplexer 172 including capacitors 178 and 179 and resistor 180, and the inputs of the multiplexers 174 and 176 further including a resistor 181. The multiplexers 172, 174 and 176 are controlled by output timing signals of the timing circuit of Fig. 2. A further multiplexer 182, also controlled by the timing circuit of Fig. 2, selectively grounds the input of the amplifier 152 and the output of the capacitor 156, in order to determine the ambient light component. Thus, when the input of the amplifier 152 is shorted to ground, in the absence of energization of the LEDs 101, 102, the capacitor 156 is charged to a level corresponding to received ambient light.

The output of the multiplexer 172, which corresponds to the component of the signal, is applied to a variable gain amplifier 190, this signal then being applied to a demultiplexer 192 controlled by the timing circuit of Fig. 2. The output of the demultiplexer 192 has two identical channels 194, 196 each including a third-order low-pass filter 198 and a sample-and-hold circuit 200. The outputs of the sample-and-hold circuits are applied as separate inputs to a multiplexer 202. The sample-and-hold circuits are controlled by a further timing signal from the timing circuit of Fig. 2. The demultiplexer 192 is controlled to pass signals to the channel 194 only during times that the red LED 101 is energized, and to pass signals to the channel 196 only during times that the infra-red LED 102 is energized.

The gain of the amplifier 190 is controlled by a multiplexer 210 connected to selectively connect input resistors 212 of different resistance to the input of the amplifier 190, the multiplexer 210 being controlled by digital control signals applied to the control lines 214 from the digital portion of the system, as will be discussed in the following paragraphs. It is to be noted that the variable gain amplifier 190 controls only the AC signals, while the variable gain amplifier 152 controls the amplitude of all signals. Accordingly, the relative levels of the DC and AC signals can be controlled by controlling the multiplexer 158 to achieve the desired DC gain and controlling the multiplexer 210 to achieve the desired AC gain.

The multiplexers 174 and 176 are controlled by the timing circuit of Fig. 2 to provide outputs only during the energization of the red and infra-red LEDs 101, 102 respectively, these outputs being smoothed by output capacitors 220, 222 respectively, and applied as separate inputs to the multiplexer 202 by way of buffers 224, 226 respectively.

The red and infra-red LEDs 101, 102 are driven by transistor drive amplifiers 250, 252 respectively, these transistor amplifiers being controlled by the output of red and infra-red digital-to-analog converters 254, 256 respectively. Data input signals from the digital portion of the circuit shown in Fig. 3 are applied to the digital-to-analog circuits 254, 256, in order to enable control of the timing and amplitude of the drive signals from the transistor amplifiers. Accordingly, the digital control circuit determines the timing and intensity of the energization of the red and infra-red LEDs. The outputs of the transistor amplifiers 250, 252 are also applied to demultiplexers 260, 262, respectively, which are also controlled by the timing circuit of Fig. 2. The outputs of the demultiplexers 260, 262, in addition to being controlled by the timing circuit, are also selectively applied to the multiplexer 202 by way of different valued resistors.

The resistor 104 of the probe is also connected to the multiplexer 202 by way a buffer amplifier 270, in order to enable the application of a signal to the system corresponding to the value of the resistor, and hence the characteristics of the probe, so that the program invoked by the system may correspond to the probe in use.

The analog circuit of Fig. 1 further includes a heater control circuit 290, responsive to the output of the temperature sensing element 106 for controlling the current applied to the heater 105, to maintain the temperature of the probe constant, for example, at 40° C.

The multiplexer 202 is controlled by the digital circuitry, by way of control lines 292. The selected output of the multiplexer 202 is applied to a buffer 294, and then to analog-to-digital converter 296, for example, a 12-bit analog-to-digital converter, for producing digital signals for application to the digital circuit of the system as illustrated in Fig. 3.

In the timing circuit of Fig. 2, the system clock having a frequency of 3.6864 MHz is applied to a divider circuit comprised of dividers 300 and 302, the multiplexer 304 and binary counter-divider 306, to produce sequential addressing signals for addressing ROM 308. The data outputs of the ROM 308 are applied to an octal D-type flip-flop 310 to latch the control signals A-F as employed in the circuit of Fig. 1. The system of Fig. 2 further produces the WCLK, DATVAL, and NVI signals for application to the digital circuit. In addition, the timing of the circuit of Fig. 2 is controlled by a FREQOUT signal applied to the multiplexer 304, to control the timing as a function of the power supply frequency as will be discussed. It is of course apparent that other timing control circuits may be alternatively employed, the system of Fig. 2 providing the advantage that the timing may be readily modified as desired in accordance with the programming of the ROM 208.

The digital control portion of the system is illustrated in the block diagram of Fig. 3, wherein the Z8002 microprocessor 400 is controlled in a conventional manner by the clock circuit 402. The address outputs of the microprocessor are latched by the address latches 404, to address ROMs 406 and 408, RAMs 410 and 412, and decoders 414 and 416. The data lines of the microprocessor system are connected to the above-noted ROMs and RAMs, as well as to a UART 420, latches 422, 424, and 426, and digital-to-analog converters 428 and 430. The output of the 12-bit analog-to-digital converter 296 of Fig. 1 is also applied to the data bus, and the data bus signals are applied to the digital-to-analog converters 254 and 256. The data bus is further connected to the display panel.

The UART 420 is employed to enable serial communication between the oximeter and external devices, such as displays, recorders, etc. and for this purpose the UART may be connected to any conventional serial interface 440. The UART clock 442, which may be comprised of dividers, is synchronized by the 3.6864 MHz system clock. The latch 422 is connected to a switch input circuit 450, comprised of a plurality of control switches on the display panel, in order to enable control of various parameters of the system. Various other system signals are applied to the latch 422 as illustrated in the Figure. The latch 424 is connected to a power supply status circuit 452, in order to enable the microprocessor to determine various power supply conditions, such as low voltage, etc. The decoder 414 provides various addressed control signals for the display panel as well as for the audio circuit, and for clocking the latches 426. The decoder 416 provides the addressed enable signals for the ROMs and RAMs.

The latches 426 provide the control signals for the gain control multiplexers 158 and 210 of the analog circuit, as well as the control signals for the audio channel and the test signal for controlling the multiplexer 154.

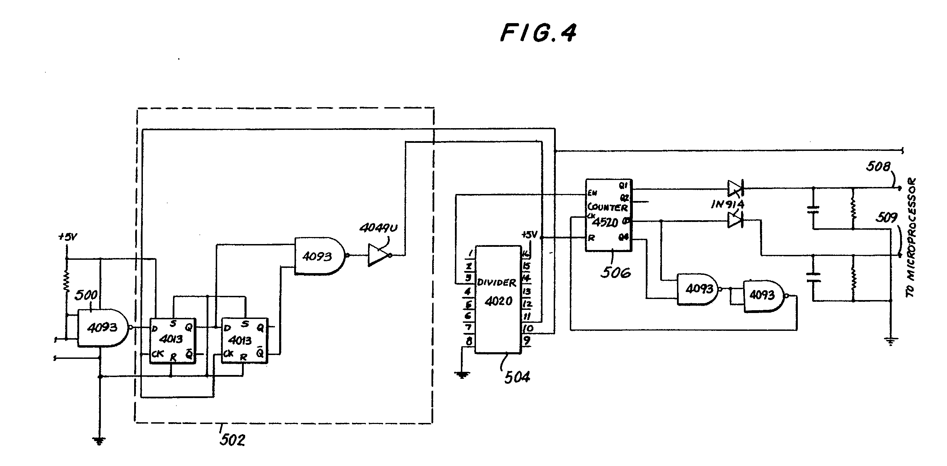

A circuit as illustrated in Fig. 4 is provided in order to detect the frequency of the power source (e.g. either 50 or 60 Hz), in order to enable determined functions of the system to occur only in synchronization with the power frequency, thereby minimizing interference at the power frequency. For this purpose, a Schmitt trigger 500 is connected to receive a low voltage signal at the power frequency (e.g. a low voltage in the conventional power supply circuit). The output of the Schmitt trigger 500 is synchronized to the system clock by circuit 502, the output of the circuit 502 being connected to a ripple carry divider 504. The system clock of 3.6864 MHz is applied to the clock terminal of the divider 504, one output of the divider 504 being applied to the enable terminal of counter 506. Two outputs of the counter 506, at terminals 508 and 509, have levels dependent upon the frequency of the power source. These outputs terminals are applied as inputs to the microprocessor, which in turn controls an output to the multiplexer 304 of the timing circuit (Fig. 2), in order to ensure the proper timing (e.g., of the sample-and-hold oontrol) in dependence upon power line frequency.

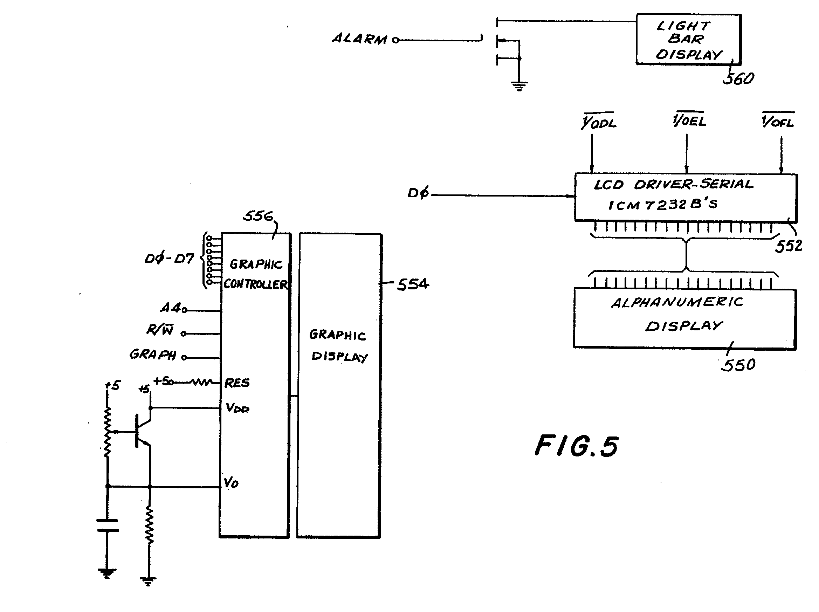

The system is further provided with a number of displays, as illustrated in Fig. 5. The system thus is provided with an alphanumeric display 550 having, e.g., 18 digits, 6 large digits and 12 smaller digits, the signals for energizing the display 550 being derived serially from the data line DO applied to conventional LCD drivers 552, e.g. two 10-1/2 digit drivers of type ICM7232B. The control signals for these drivers are obtained, e.g., from the output of the selectors of the digital control circuit.

The display in accordance with the invention is further comprised of a graphic display in the form of an LCD matrix display 554, e.g., of type LM51B32G120DB. The graphic display 554 is controlled by controller 556, e.g., of type LM1001GC, which receives the data signals D0-D7 as well as various further control signals, as indicated, from the digital control circuit above discussed. The graphic display, as will be disclosed in greater detail in the following paragraphs, enables the display of various data obtained from the oximeter signals.

The display panel further comprises a light bar display 560 connected to be actuated in the event of an alarm.

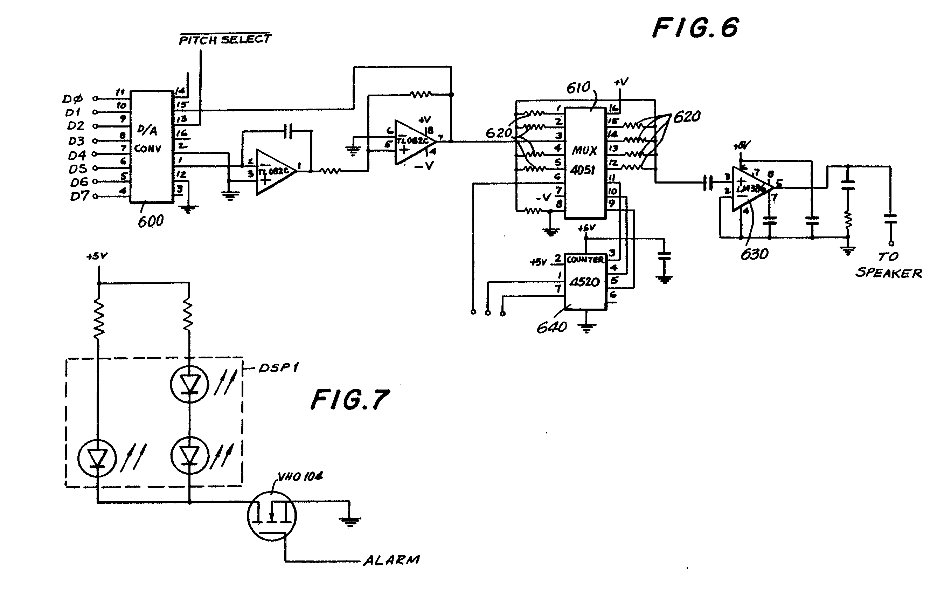

It has been found that variations of pitch of an alarm signal provide advantageous results in the operation of the oximeter, and for this purpose a variable pitch alarm circuit has been provided as illustrated in Fig. 6. In this arrangement the data signals D0-D7 of the digital control circuit are applied to a digital-to-analog converter 600 to form an analog signal of the desired frequency corresponding to oxygen saturation. This signal is supplied as an input to multiplexer 610, the multiplexer being controlled to insert determined resistors 620 in series with the signal before it is applied to the output amplifier 630. The multiplexer 610 is controlled by a counter circuit 640 which sequences the outputs of the multiplexer to provide a varied amplitude output. The alarm signal applied to the speaker (not shown) may hence be controlled in pitch and amplitude in accordance with the program of the microprocessor.

Various other alarms may be provided for the system, such as the visual alarm as illustrated in Fig. 7 which is controlled by the alarm signal.

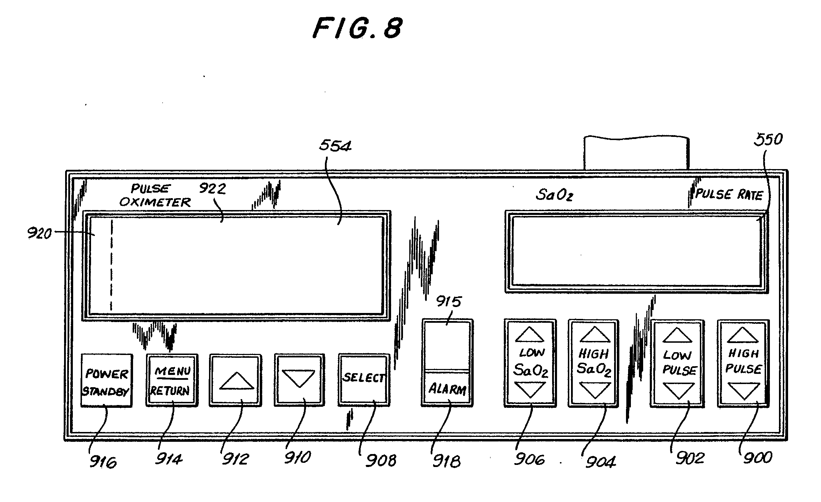

One embodiment of a display panel for an oximeter, in accordance with the invention, is illustrated in Fig. 8. this display panel incorporates the alphanumeric display 550 and the graphic display panel 554. In addition, a number of switches 900, 902, 904, 906, 908, 910, 912, 914 and 915 are provided, corresponding to the switch inputs 450 of the digital control circuit of Fig. 3. Of these switches, the switches 900, 902, 904 and 906 may constitute double switches, in order that signals representing two different conditions can be sent to the microprocessor. As discussed above, the outputs of these switches are coupled to the latch 422 of Fig. 3, to enable the microprocessor to test the conditions of the signals at determined times and to make the appropriate response thereto.

The panel further includes a power standby switch 916 of conventional design, and an alarm indicator 918 which may be of the form previously discussed.

The microprocessor circuit is programmed to calculate the quantity $SaO_2$, in accordance with the relationships discussed in U.S. Patent No. 4,407,290, issued to Wilber, and assigned to the assignee of the present application. These quantities are applied to the data bus, specially to data line DO, and directed serially to the drivers of the alphanumeric display 550. In a preferred embodiment, e.g., the left-hand large digits of the display 550 display the current $SaO_2$ level and the right-hand large digits display the calculated pulse rate. It is noted that these quantities are also available serially from the serial interface 440 of Fig. 3, for application to any other desired external equipment.

In order to enable the setting of upper and lower limits of $SaO_2$ level and pulse rate, at which limits it is desired to provide a visual or audible alarm, the switches 900, 902, 904 and 906, corresponding to the high limit of the pulse rate, the low limit of the pulse rate, the high limit of $SaO_2$, the low limit of $SaO_2$ respectively, have upper switch positions which, when depressed, raise the respective limit value and lower switch positions which, when depressed, lower the respective limit value. For example, the microprocessor may be responsive to depression of the upper portion of the switch 906 for raising the low limit of $SaO_2$, and responsive to depression of the lower portion of the switch 906 for lowering the low limit of $SaO_2$. The limits that have been selected may be visually observed on the display 550 in sets of three small digits located above the respective limit switches 900, 902, 904 and 906. When the measured quantities $SaO_2$ or pulse rate exceed these high or low limits, the microprocessor provides an alarm signal, to energize one or more of the above-discussed alarms.

The graphic display panel 554 is divided into two areas 920 and 922. The area 920, which constitutes a signal column or bar graph at the left side of the display, is a "perfusion" indicator. The microprocessor is programmed to display a vertical bar in the region 920 of the display 554, having a height corresponding to the average intensity of the AC component of the signal. This display then enables the approximate visual determination of the amplitude of the AC signals. If the AC signals are too low, resulting in a low value or disappearance of the bar graph, it is apparent to an observer that inadequate signals have been applied to the equipment to enable valid measurement. Accordingly, the observer may readily determine if the signal strength is adequate for valid measurements. It is of course apparent that other types of bar graphic displays may be employed for this purpose such as conventional bar graph indicators. The important aspect of this concept of indication, however, lies in the display of a quantity enabling determination by the observer of the amplitude of the AC components of the signal, in order to avoid reliance upon potentially invalid measurements.

The right-hand portion 922 of the graphic display forms a non-diagnostic plethysmographic display. In accordance with the invention, the input signals are sampled thirty times a second (employing a 60 cycle power frequency). The amplitude of the sensed signal is displayed each time at the left-hand column of the displayed area 922, with the display continually moving to the right upon reception of each new sample signal. Accordingly, the displayed area 922 clearly displays a moving plethysmographic pattern similar to a non-diagnostic EKG, but which is not sufficiently accurate for diagnostic purposes. This non-diagnostic display serves the purpose of providing the observer with a further indication of other conditions that may render the oximeter reading invalid. For example, variations that may occur as a result of movement of the patient, coughing of the patient, etc., result in variations in the non-diagnostic display which are obvious upon observing the display portion 922, so that measurements of $SaO_2$ in the presence of such motion may be readily discarded by the observer as invalid.

The graphic display in the display portion 922 may be effected by conventional techniques, with the amplitude of the bar in each column being displaced to the next column to the right upon receipt of each new signal. It is of course apparent that this portion of the display may alternatively be fabricated of a series of conventional bar graph displays assembled side by side.

The plethysmographic display produced in the display portion 922 is of a shape similar to that of the conventional non-diagnostic EKG employed medically, e.g., in operating rooms. This similarity enables the interpretation of the plethysmographic display without difficulty by those familiar with non-diagnostic EKG displays. It has not heretofore been realized that the validity of oximeter readings may be impaired by patient movement, coughing or the like. The display in accordance with the invention enables determination of reading validity without difficulty and facilitates the acceptance of only valid data. Prior art devices neither recognized the presence of invalid data nor provided a manner for ignoring such data.

In operation, the probe 100 is placed in a measuring position against living tissue, such as a finger or ear lobe. The program of the system selectively energizes the red and infra-red LEDs 101, 102 at different times, the digital-to-analog converters 254, 256 controlling the intensity of energization in order to ensure that the emitted light is within a permissable range allowing satisfactory sensing of signals by the photosensing diode 103. The value of the resistor 104 is sensed in order to provide data for the microprocessor so that the characteristics of the probe are taken into consideration in the calculations.

The sequencing of the energization of the red and infra-red LEDs may be effected, for example, by energizing them one after the other, followed by a period during which both of them are dark. The dark period enables establishment of the ambient conditions, so that the effect of such ambient conditions may be removed from the measurement results.

The gains of the variable gain amplifiers 152 and 190 are controlled by the multiplexers 158 and 210, as above discussed. Thus, signals corresponding to the AC and DC quantities at each wavelength are applied to the multiplexer 202. The multiplexers 260 and 262, whose outputs are also applied to the multiplexer 202, enable determination by the microprocessor of the current intensity level of the respective LEDs. Since the multiplexer 202 is controlled by the digital control circuit of Fig. 3, by way of the latches 426, it is apparent that the program of the microprocessor may select these signals for processing in any desired sequence. The analog-to-digital converter 296 converts the measured analog signals to digital form for processing in the microprocessor.

The timing circuits of Fig. 2, controlling various multiplexers of the analog circuit of Fig. 1, follow a sequence determined by the ROM 208, the timing control signals being synchronized with the system clocks of the digital circuitry. Since it is desirable to perform certain functions, such as those performed by the sample-and-hold circuits 200, in synchronization with the power frequency in order to avoid interference, the timing circuit of Fig. 2 has an input to the divider 204, ensuring such timing of the control of the sample-and-hold circuits.

The actual calculations, employing the algorithms disclosed by Wilber for determination of SaO₂, are effected by the program of the microprocessor, which is stored in the ROMs 406 and 408. The calculation quantities are applied by the program to the data bus for direction to the display panel and the serial interface. The program of the microprocessor receives the digital output signals of the analog-to-digital converter 296 on 12 bits of the data bus for processing. The control signals for the gain control of variable gain amplifiers 156 and 190 by multiplexers 158 and 210, as well as for the multiplexer 202, are also derived from the data bus.

The program of the processor effects normalization of the AC signals by taking the ratios $AC_i/DC_i$, where i = 1 to n, and n is the number of wavelengths used.

The energization of the LEDs may be effected at the rate disclosed in the Wilber patent, preferably employing only one dark time for each cycle.

## Claims

1. An oximeter including means for transmitting light of at least two different wavelengths through blood-containing tissue, sensing means (103) for transducing said transmitted light of said different wavelengths into signals, means producing a plurality of signals corresponding to the attenuation of said transmitted light at the different wavelengths, data processing means responsive to said signals for determining the oxygen saturation level (SaO₂) of blood in said tissue, and digital display means for displaying said oxygen saturation level; is characterised in that said display means further comprises an LCD matrix display (554), said data processing means including means for applying signals to the matrix display (554) for displaying a plethysmographic waveform and further means for controlling said matrix display (554) to display thereon a bar having an amplitude corresponding to the average amplitude of the AC component of said signals.

## Revendications

1. Oximètre comprenant des moyens destinés à transmettre de la lumière sous au moins deux longueurs d'onde différentes à travers un tissu contenant du sang, des moyens détecteurs (103) destinés à convertir en signaux la lumière transmise sous lesdites longueurs d'onde différentes, des moyens destinés à produire plusieurs signaux correspondant à l'atténuation de ladite lumière transmise sous les longueurs d'onde différentes, des moyens de traitement d'information sensibles auxdits signaux pour déterminer le niveau de saturation en oxygène (SaO₂) du sang dans ledit tissu, et des moyens d'affichage numériques destinés à afficher ledit niveau de saturation en oxygène; caractérisé en ce que lesdits moyens d'affichage comprennent en outre un afficheur matriciel à cristaux liquides (554), lesdits moyens de traitement d'information comprenant des moyens destinés à appliquer des signaux à l'afficheur matriciel (554) afin d'afficher une forme d'onde plethysmographique, et en outre des moyens destinés à commander ledit afficheur matriciel (554) afin d'afficher sur celui-ci une barre ayant une amplitude correspondant à l'amplitude moyenne de la composante alternative desdits signaux.

**Ansprüche**

1.  Ein Oximeter, das enthält Mittel zum Aussenden von Licht von mindestens zwei verschiedenen Wellenlängen durch Blut enthaltendes Gewebe, Fühlermittel (103) zum Wandeln des ausgesendeten Lichts mit den unterschiedlichen Wellenlängen in Signale, eine Vielzahl von der Schwächung des ausgesendeten Lichts bei den unterschiedlichen Wellenlängen entsprechenden Signalen erzeugendes Mittel, auf die Signale zur Bestimmung des Sauerstoff-Sättigungspegels ($SaO_2$) des Blutes in dem Gewebe reagierendes Datenbearbeitungsmittel und Digital-Anzeigemittel zum anzeigen des Sauerstoff-Sättigungspegels; ist dadurch gekennzeichet, daß das Anzeigemittel weiter eine Flüssigkristall-Matrixanzeige (554) umfaßt, wobei das Datenbearbeitungsmittel Mittel zum Anlegen der Signale an die Matrixanzeige (554) zum Anzeigen eines plethysmographischen Wellenzuges enthält und weiter Mittel zum Steuern der Matrixanzeige (554), um daran einen Strich mit einer der durchschnittlichen Wechselspannungskomponente des Signals entsprechenden Amplitude anzuzeigen.

*FIG.1*

PROBE

ANALOG CIRCUITS

PHOTO SENSE DIODE 103
100
105

HEATER

TEMP. SENSING ELEMENT 106

LOW NOISE AMP 150

3Hz TEST SINE WAVE GEN 164

HEATER CONTROL CIRCUIT 290

MULTIPLEXER 4053 154
AD TEST 166

MULTIPLEXER 4053 158
DIGITAL DC GAIN CONTROL SIGNALS 160 162

VARIABLE GAIN 152 156

SWITCHED LOW-PASS FILTER 151

MUX 4053 182
AAE

VARIABLE GAIN

MUX 4053 172
CCE
180 178 179

MULTIPLEXER 4053 210 212
DIGITAL AC GAIN CONTROL SIGNALS 214

D MUX 4053 190 192
EC

3rd ORDER LOW PASS FILTER 198
3rd ORDER LOW PASS FILTER 198

SAMPLE AND HOLD 200 194
SAMPLE AND HOLD 200
196 F

MUX 4051 202
292
DIGITAL OUTPUT CONTROL

12 BIT A/D HS 574 296
294

MUX 4053 174 181 220
224

MUX 4053 176 222
226

D MUX 4053 260
BCD 250

D MUX 4053 262 252
B E D

RED DRIVE D/A D0-D7 254
270 VREF

D/A 7524 256
D0 D1 D2 D3 D4 D5 D6 D7
VREF WR

101
102
104

8

FIG.2

FIG.3

FIG.4

FIG.5

FIG.6

FIG.7

FIG.8